# EUROPÄISCHE PATENTSCHRIFT

(11) **EP 2 452 172 B1**
(45) Veröffentlichungstag und Bekanntmachung des Hinweises auf die Patenterteilung: **05.09.2018**
(21) Anmeldenummer: 09777096.0
(22) Anmeldetag: 10.07.2009
(51) Int. Cl.: G01K 7/04, G01K 13/00, A61B 18/00, A61B 18/16

(54) **TEMPERATURFÜHLER ZUR MESSUNG AN ODER IN EINEM LEBENDEN KÖRPER**
TEMPERATURE SENSOR FOR MEASUREMENT ON OR IN A LIVING BODY
CAPTEUR DE TEMPÉRATURE POUR LA MESURE SUR/ OU DANS UN CORPS VIVANT

(43) Veröffentlichungstag der Anmeldung: 16.05.2012
(73) Patentinhaber: Stockert GmbH, 79111 Freiburg (DE)
(72) Erfinder: Stockert, Rüdiger, 79104 Freiburg (DE)
(74) Vertreter: Katérle, Axel
(86) Internationale Anmeldenummer: PCT/EP2009/005012
(87) Internationale Veröffentlichungsnummer: WO 2011/003432

(56) Entgegenhaltungen:
- EP-A1- 1 172 070
- EP-A2- 1 990 020
- US-A- 1 715 316
- US-A- 5 033 866
- US-A- 5 288 147
- US-A1- 2007 049 919
- US-A1- 2008 195 089

## Beschreibung

Die Erfindung betrifft einen Temperaturfühler zur Temperaturmessung an oder in einem lebenden Körper, insbesondere zur Verwendung bei der Thermoenergie.

Bei der Thermoenergie kommt es immer wieder zu Gewebeüberhitzungen oder Hautverbrennungen. So wird bei der Thermochirurgie biologisches Gewebe durch Einbringung von Behandlungsenergie erwärmt, um durch Denaturierung des behandelten Gewebes einen bestimmten therapeutischen Zweck zu erzielen. Insbesondere wird bei der Thermochirurgie eine Koagulation oder Ablation lokaler Gewebebereiche angestrebt. Beispielsweise können mittels Thermochirurgie Arrhythmien oder Tachikardien am Herzen des menschlichen oder tierischen Körpers behandelt werden. Bei einer solchen Koagulation oder Ablation am bzw. im Herz entstehen in benachbartem Gewebe (beispielsweise in der Speiseröhre) oft unerwünschte Gewebeüberhitzungen.

Bei der elektrischen Hochfrequenz-Thermochirurgie sind monopolare und bipolare Anwendungen bekannt. Bei beiden Behandlungsarten wird ein zur Behandlung dienender hochfrequenter Wechselstrom über eine Anwendungselektrode in den Körper eingespeist, die in unmittelbare Nähe des zu behandelnden Gewebebereichs gebracht wird. Bei der bipolaren Technik wird der Stromkreis über mindestens eine flächige Gegenelektrode geschlossen, die in einem Abstand von der Anwendungselektrode außen auf die Haut des Körpers aufgelegt bzw. aufgeklebt wird. Die Gegenelektrode wird auch als Neutralelektrode, indifferente Elektrode oder Return-Elektrode bezeichnet. Die wirksame Elektrodenfläche der Gegenelektrode ist groß im Vergleich zu derjenigen der Anwendungselektrode, weshalb unter normalen Umständen (d.h. gutem Kontakt der Elektrode mit der Haut) die Stromdichte an der Gegenelektrode gering ist, so dass dort keine Verkohlung der Haut zu befürchten ist.

Die Situation ändert sich, wenn sich die Gegenelektrode teilweise von der Haut ablöst. Dies kann durch Bewegung des Patienten und/oder Schweißbildung, insbesondere bei einer sich über mehrere Stunden erstreckenden Behandlung, erfolgen. In diesem Fall nimmt die Stromdichte in den noch mit der Haut in Kontakt befindlichen Bereichen der Gegenelektrode stark zu, und dementsprechend vergrößert sich die Gefahr von Hautverkohlungen.

Sowohl in den oben beschriebenen zu dem Ablations- bzw. Koagulationsort benachbarten Gewebebereichen (beispielsweise der Speiseröhre) als auch in nicht von der Haut abgelösten Bereichen der Gegenelektrode treten unerwünschte Gewebeüberhitzungen meist nur punktuell, d.h. in einem relativ kleinen Flächenbereich, auf. Bekannte Temperaturfühler zur Messung an oder in einem lebenden Körper bestimmen jedoch nur Durchschnittstemperaturwerte eines relativ großflächigen Messbereichs oder der Umgebung des Temperaturfühlers, so dass punktuelle Gewebeüberhitzungen nicht mit ausreichender Zuverlässigkeit erfasst werden können.

Beispiele von bekannten Thermopaaren zur Temperaturmessung an oder in einem lebenden Körper sind aus den Patentschriften EP1172070, US1715316, EP1990020 und US2007/0049919 zu entnehmen.

Zur Lösung des Problems der Erfassung unerwünschter punktueller Gewebeüberhitzungen ist es denkbar, eine Vielzahl von Temperatursensoren zu verwenden. Die Herstellung und Ansteuerung eines Temperaturfühlers mit einer Vielzahl von Temperatursensoren ist jedoch technisch aufwendig und entsprechend teuer.

Aufgabe der Erfindung ist es, einen Temperaturfühler zur Temperaturmessung an oder in einem lebenden Körper bereitzustellen, der zuverlässig punktuelle Gewebeüberhitzungen an oder in dem lebenden Körper erfasst und mit geringem technischen Aufwand herstellbar ist.

Zur Lösung dieser Aufgabe sieht die Erfindung einen Temperaturfühler zur Temperaturmessung an oder in einem lebenden gemäß dem Anspruch 1 vor. Bei den Thermopaaren kann es sich insbesondere um Thermoelemente handeln. Der erfindungsgemäße Temperaturfühler gestattet eine Erfassung von Temperaturänderungen an jedem der Thermoübergänge. Bei den Temperaturänderungen kann es sich sowohl um Temperaturerhöhungen als Temperaturerniedrigungen handeln. So können bei gegebenen Temperaturerhöhungen beispielsweise alle Thermoübergänge der ersten Gruppen eine positive Spannungsänderung und alle Thermoübergänge der zweiten Gruppe eine negative Spannungsänderung bewirken. Durch die Reihenschaltung können auch punktuelle Temperaturänderungen (beispielsweise nur an einem Thermoübergang) erkannt werden, da der Temperaturfühler auch bei einer lokal begrenzten Temperaturänderung einen Messwertausschlag (und nicht nur einen Durchschnittsmesswert) liefert. Durch die Aufteilung der Thermoübergänge in zwei Gruppen kann ferner erfasst werden, in welchem Bereich der Messfläche die punktuelle Temperaturänderung auftritt.

Es sind vielfältige Anordnungen von Thermoübergängen auf der Messfläche des Temperaturfühlers denkbar. Damit punktuelle Temperaturänderungen mit möglichst hoher Wahrscheinlichkeit erfasst werden, ist eine im Wesentlichen homogene Verteilung von Thermoübergängen auf der Messfläche vorteilhaft. Insbesondere können die Thermoübergänge zumindest teilweise derart auf der Messfläche angeordnet sein, dass sie auf Eckpunkten einer regulären Parkettierung liegen. Vorzugsweise können die Thermoübergänge zumindest teilweise auf Eckpunkten einer regulären Parkettierung bestehend aus gleichseitigen Dreiecken, Quadraten oder regelmäßigen Sechsecken liegen. Falls die Messfläche eine Kreiszylinderform aufweist, können die gleichseitigen Dreiecke, Quadrate oder regelmäßigen Sechsecke auf die Mantelfläche des Kreiszylinders projiziert sein. Ferner können die jeweiligen Thermoübergangsgruppen im Wesentlichen auf Eckpunkten einer regulären Parkettierung liegen.

Die Thermopaare weisen vorzugsweise unterschiedliche erste und zweite elektrische Leiter auf. So kann es sich bei den ersten und zweiten elektrischen Leitern um unterschiedliche Metalle, Legierungen oder Halbleiter mit unterschiedlichen elektrischen Leitfähigkeiten handeln. Zur einfachen Herstellung der Thermopaare bzw. der Gruppierungen kann der Temperaturfühler eine Schichtstruktur mit mindestens einer ersten und einer zweiten Schicht aufweisen, wobei die ersten elektrischen Leiter in der ersten Schicht und die zweiten elektrischen Leiter in der zweiten Schicht liegen und sich die Thermoübergänge zumindest teilweise zwischen der ersten und der zweiten Schicht erstrecken.

Vorzugsweise sind die erste und die zweite Schicht im Wesentlichen parallel zueinander, wobei sich die Thermoübergänge zwischen der ersten und der zweiten Schicht erstrecken. Insbesondere können sich die Thermoübergänge im Wesentlichen senkrecht zu der ersten und der zweiten Schicht erstrecken. In Abhängigkeit von dem eingesetzten Fertigungsverfahren können sich Teile der ersten und/oder der zweiten elektrischen Leiter in dem Erstreckungsbereich zwischen der ersten und zweiten Schicht befinden. Dadurch wird eine einfach zu fertigende, dreidimensionale Struktur bestehend aus den ersten und zweiten elektrischen Leitern und den Thermoübergängen bereitgestellt. Vorzugsweise befindet sich die Schichtstruktur auf einen die Messfläche bildenden Körper, beispielsweise einem Hohlkörper.

In einer bevorzugten Ausführungsform liegen die ersten Leiter überlappungsfrei in der ersten Schicht und die zweiten Leiter überlappungsfrei in der zweiten Schicht. Dadurch können alle ersten und zweiten elektrischen Leiter in jeweils einem Fertigungsschritt hergestellt werden. Zwischen den ersten und zweiten elektrischen Leitern kann ferner zumindest teilweise eine elektrisch isolierende Schicht angeordnet sein. In diesem Fall ist die elektrisch isolierende Schicht in der Schichtstruktur zwischen der ersten und zweiten Schicht angeordnet.

Um eine hohe Anzahl bzw. eine große Dichte von Thermoübergängen pro Flächeneinheit zu ermöglichen, können sich die in der ersten und zweiten Schicht liegenden elektrischen Leiter gegenseitig teilweise überlappen.

Gemäß einer bevorzugten Ausführungsform sind die ersten und zweiten elektrischen Leiter und die Thermoübergänge auf einer flexiblen Leiterplatte angeordnet. Dadurch wird eine Anpassung der Leiterstruktur an eine vorgegebene Messflächenform vereinfacht. Die flexible Leiterplatte kann insbesondere auf die Messfläche des Temperaturfühlers aufgeklebt werden.

Zur genaueren Lokalisierung von Temperaturänderungen können die Thermopaare elektrisch zu mindestens zwei Reihenschaltungen verbunden sein. So können die von den in den mindestens zwei Reihenschaltungen verbundenen Thermopaare gebildeten Thermoübergänge pro Reihenschaltung auf mindestens zwei räumlich voneinander getrennte Gruppen aufgeteilt sein, derart, dass in jeder Gruppe die Thermoübergänge bei einer gegebenen Temperaturänderung eine jeweils gleichsinnige Spannungsänderung bewirken. Dadurch können Temperaturänderungen in mindestens vier räumlichen Bereichen erfasst werden. Entsprechend kann durch weitere Reihenschaltungen die Lokalisierungsgenauigkeit von Temperaturänderungen gesteigert werden.

Um auch eine punktuelle Temperaturänderung an einem Ort zwischen den beiden Thermoübergangsgruppen der ersten Reihenschaltung mit hoher Sicherheit bestimmen zu können, kann zwischen mindestens zwei in elektrischer Reihe verbundenen Thermoübergängen mindestens ein Thermoübergang einer anderen Reihenschaltung angeordnet sein. Falls beispielsweise die Thermoübergänge der ersten Gruppe bei einer Temperaturerhöhung eine positive Spannungsänderung bewirken und die Thermoübergänge der zweiten Gruppe bei einer Temperaturerhöhung eine negative Spannungsänderung bewirken, so können sich bei einer punktuell zwischen den beiden Gruppen auftretenden Temperaturänderung die beiden Spannungsänderungen zu Null aufheben, so dass der Temperaturfühler trotz einer Temperaturerhöhung keinen von Null verschiedenen Messwert liefert. Durch die Anordnung mindestens eines Thermoübergangs zwischen den beiden Thermoübergangsgruppen der ersten Reihenschaltung kann eine solche punktuelle Erwärmung jedoch erfasst werden.

Um eine hohe Anzahl bzw. eine große Dichte von Thermoübergängen pro Flächeneinheit zu ermöglichen, können die Thermopaare der mindestens zwei Reihenschaltungen gegenseitig teilweise überlappend auf der Messfläche angeordnet sein. Alternativ können die Thermopaare der mindestens zwei Reihenschaltungen auch gegenseitig überlappungsfrei auf der Messfläche angeordnet sein.

Nicht alle Thermopaare müssen auf der Messfläche angeordnet sein. So können Thermoübergänge mindestens einer Gruppe zumindest teilweise entlang einer Geraden und Thermoübergänge mindestens einer weiteren Gruppe zumindest teilweise auf der Messfläche um die Gerade angeordnet sein. Beispielsweise kann die Messfläche eine Zylinderform, insbesondere eine Kreiszylinderform, aufweisen, wobei die Thermoübergänge der einen Gruppe auf einer sich durch den Mittelpunkt des Zylinders erstreckenden Geraden liegen und die Thermoübergänge der weiteren Gruppe auf der Mantelfläche des Zylinders angeordnet sind. Eine solche längliche Form kann bevorzugt bei Speiseröhrensensoren eingesetzt werden.

Nutzer von Temperaturfühlern sind gewohnt, dass der Temperaturfühler bei Temperaturerhöhungen einen erhöhten Messwert liefert. Wie vorstehend beschrieben kann der erfindungsgemäße Temperaturfühler bei Temperaturerhöhungen jedoch auch negative Messwerte liefern. Zur Vermeidung dieses Problems kann der Temperaturfühler eine elektrische Schaltung aufweisen, welche den absoluten Betrag des Messwerts liefert. Bei mehreren Reihenschaltungen kann die elektrische Schaltung auch absolute Beträge von Spannungsmesswerten der mehreren Reihenschaltungen bilden.

Gemäß einer bevorzugten Ausführungsform kann der Temperaturfühler zusätzlich einen Temperatursensor zum Messen eines Temperaturwerts aufweisen. Bei dieser Ausführungsform kann die elektrische Schaltung dazu eingerichtet sein, den von dem Temperatursensor gemessenen (absoluten) Temperaturwert und den absoluten Betrag des Messwerts zu addieren, so dass dem Nutzer des Temperaturfühlers eine relative Temperaturerhöhung hinsichtlich des von dem Temperatursensor gemessenen Temperaturwerts bereitgestellt wird. Falls die Messfläche die Form eines Zylinders aufweist, kann der Temperatursensor vorzugsweise innerhalb der Messfläche angeordnet sein, wobei sich die Thermopaare auf der Messfläche um den Temperatursensor befinden.

Der erfindungsgemäße Temperaturfühler kann vorzugsweise bei einem Speiseröhrensensor gemäß Anspruch 14 verwendet werden. Gemäß einer bevorzugten Ausführungsform weist der Speisenröhrensensor eine längliche, kreiszylindrische Form auf, wobei die Thermopaare zumindest teilweise an der Mantelfläche des Kreiszylinders angeordnet sind. Die Länge des Speiseröhrensensors ist an die Länge der zu messenden Speisenröhre angepasst. Dazu kann der Speisenröhrensensor in der Länge veränderbar (beispielsweise durch eine Teleskopvorrichtung) ausgebildet sein.

Die Erfindung wird nachfolgend anhand der beigefügten Zeichnungen weiter erläutert. Es stellen dar:
- Figur 1: eine schematische Darstellung einer Reihenschaltung von Thermoelementen;
- Figur 2: eine schematische Darstellung eines Temperaturfühlers;
- Figur 3: eine schematische Darstellung einer ersten Leiterstruktur eines Ausführungsbeispiels eines erfindungsgemäßen Temperaturfühlers;
- Figur 4: eine schematische Darstellung einer zweiten Leiterstruktur des Ausführungsbeispiels;
- Figur 5: eine schematische Darstellung einer Überlagerung der ersten und zweiten Leiterstruktur der Figuren 3 und 4;
- Figur 6: eine seitliche schematische Darstellung des erfindungsgemäßen Temperaturfühlers gemäß dem Ausführungsbeispiel;
- Figur 7: eine schematische Darstellung eines ersten Ausführungsbeispiels eines erfindungsgemäßen Speisenröhrensensors;
- Figur 8: eine schematische Ansicht von oben des Speiseröhrensensors der Figur 7; und
- Figur 9: eine schematische Darstellung eines zweiten Ausführungsbeispiels eines erfindungsgemäßen Speisenröhrensensors.

Gleiche oder gleichwirkende Komponenten sind in den Figuren durch gleiche Bezugszeichen bezeichnet.

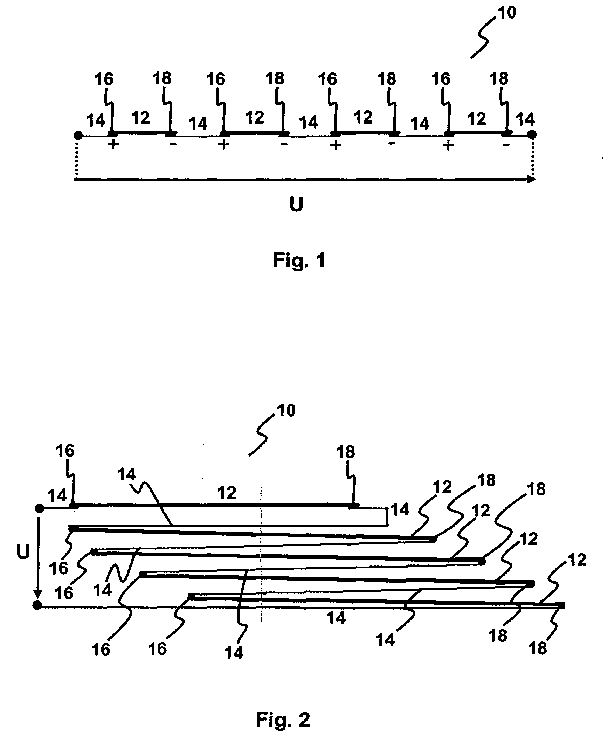

Anhand der in dem Beispiel der Figur 1 allgemein mit 10 bezeichneten Reihenschaltung wird ein für die vorliegende Erfindung grundlegendes Prinzip erläutert. Die Reihenschaltung 10 besteht aus einer abwechselnden elektrischen Reihenschaltung von ersten 12 und zweiten 14 elektrischen Leitern. Bei den elektrischen Leitern 12 und 14 handelt es sich um unterschiedliche Metalle, Legierungen oder Halbleiter. An den Verbindungsstellen zwischen den Leitern 12 und 14, d.h. den Leiterübergängen 16 und 18, sind Thermoübergänge gebildet.

Bei einer gegebenen Temperaturerhöhung liefern die Thermoübergänge 16 eine positive Spannungsänderung, wohingegen die Thermoübergänge 18 bei einer gegeben Temperaturerhöhung eine negative Spannungsänderung liefern. Weisen alle Thermoübergänge 16 und 18 die gleiche Temperatur auf, so ist die gemessene Spannung U gleich Null. Erfolgt punktuell an einem der Thermoübergänge 16 eine Temperaturerhöhung, so wird die Spannung U positiv, wohingegen bei einer punktuellen Temperaturerhöhung an einem der Thermoübergänge 18 eine negative Spannungsänderung U erfolgt. Auch bei einer nur punktuellen Temperaturänderung liefert die Schaltung 10 einen klar erkennbaren Spannungsausschlag, d.h. eine Spannung U mit voller Amplitude. Dies liegt daran, dass nur punktuell auftretende Temperaturerhöhungen nicht durch eine Mittelwertbildung unterdrückt werden.

Die Figur 2 zeigt eine schematische Darstellung eines weiteren Beispiels eines Temperaturfühlers. Die Reihenschaltung 10 der Figur 2 unterscheidet sich von der Reihenschaltung 10 der Figur 1, in dem die Thermoübergänge 16 und 18 in zwei räumlich voneinander getrennte Gruppen aufgeteilt sind. Dabei sind die Thermoübergänge 16 jeweils links und die Thermoübergänge 18 jeweils rechts gruppiert entlang zweier zueinander paralleler Geraden angeordnet. Die gezeigte Anordnung ist jedoch nur beispielhaft. Die Thermoübergänge 16 und 18 können beispielsweise auch entlang paralleler Kreise, paralleler Kreisbögen, paralleler Spiralen oder paralleler Spiralenbögen angeordnet sein.

Mit der in der Figur 2 gezeigten Anordnung ist eine grobe Lokalisierung hinsichtlich des Bereichs der Temperaturerhöhung möglich. Eine solche grobe Lokalisierung ist für viele Anwendungsgebiete des erfindungsgemäßen Temperaturfühlers ausreichend, da der Nutzer oft nur signalisiert bekommen will, ob eine Temperaturerhöhung auftritt. Bei einem Einsatz des erfindungsgemäßen Temperaturfühlers in einem Speiseröhrensensor will der Nutzer oft nur die Information erhalten, dass eine unerwünschte Gewebeüberhitzung auftritt, worauf er die thermochirurgische Behandlung abbrechen kann.

Die Reihenschaltung 10 der Figur 2 kann auch an einer Seite einer flächigen Gegenelektrode für die Thermochirurgie (nicht gezeigt) angebracht werden. Zur Vermeidung von Hautverkohlungen kann durch die Reihenschaltung 10 erfasst werden, ob sich die Gegenelektrode von der Haut abgelöst hat. Wird die Reihenschaltung 10 wie in der Figur 2 gezeigt auf eine Seite einer Gegenelektrode angebracht, so wird dem Nutzer der Gegenelektrode bei einer positiven Spannungserhöhung signalisiert, dass sich im linken Bereich der Gegenelektrode eine Temperaturerhöhung eingestellt hat. Entsprechend wird dem Nutzer bei einer negativen Spannungserhöhung signalisiert, dass sich im rechten Bereich der Gegenelektrode eine Temperaturerhöhung eingestellt hat. Folglich hat sich bei einer positiven Spannungsänderung U der rechte Bereich und bei einer negativen Spannungsänderung U der linke Bereich der Gegenelektrode von der Haut abgelöst.

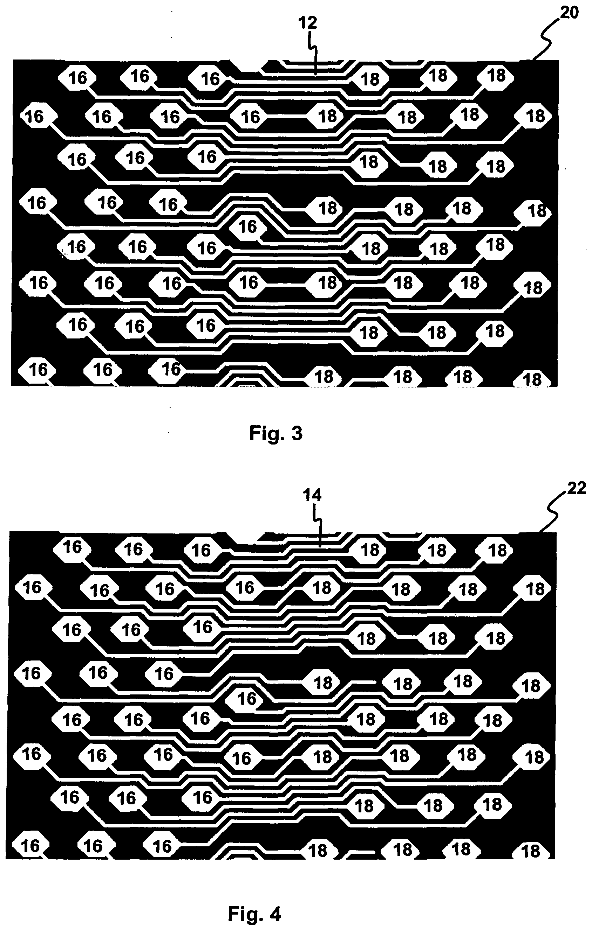

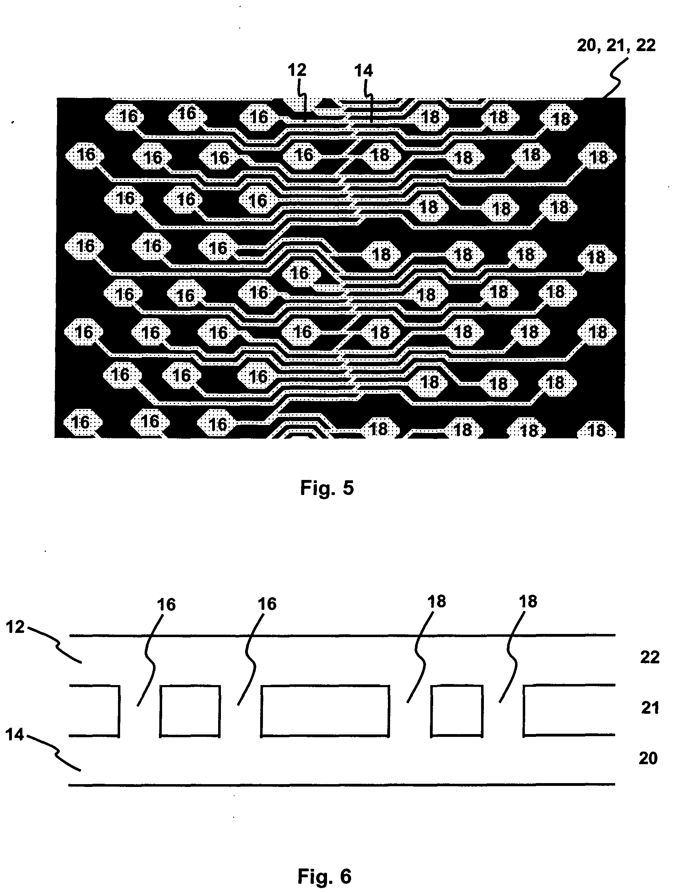

Die Figuren 3 bis 6 zeigen in schematischen Darstellungen ein Ausführungsbeispiel eines erfindungsgemäßen Temperaturfühlers. Dabei zeigt die Figur 3 eine in einer ersten Ebene angeordnete erste Leiterstruktur, die Figur 4 eine in einer zweiten Ebene angeordnete zweite Leiterstruktur, die Figur 5 übereinander angeordnet die erste und die zweite Leiterstruktur und die Figur 6 eine seitliche Schnittansicht der ersten und zweiten Leiterstruktur.

Aus der Figur 3 ist die in der ersten Ebene 20 angeordnete erste elektrische Leiterstruktur ersichtlich. Dabei ist jeweils ein Thermoübergangsbereich 16 über jeweils eine elektrische Leiterbahn 12 mit einem Thermoübergangsbereich 18 verbunden. Die Leiterbahnen 12 sind nicht überlappend in der ersten Ebene 20 angeordnet. Die Leiterbahnen 12 sind dünn ausgebildet, um Platz für möglichst viele Thermoübergangsbereiche 16 und 18 bereitzustellen. Dem entgegen weisen die Thermoübergangsbereiche 16 und 18 eine flächige, sechseckige Form auf, um auf einfache Weise mit in einer anderen Ebene liegenden Thermoübergangsbereichen 16 und 18 elektrisch verbunden werden zu können. Die sechseckige Thermoübergangsbereiche 16 und 18 sind nur beispielhaft gezeigt. Andere, insbesondere flächige Formen, sind denkbar. Alle in der Figur 3 gezeigten elektrischen Leiter 12 und Thermoübergangsbereiche 16 und 18 bestehen aus dem gleichen Metall.

Die Figur 4 zeigt die in der zweiten Ebene 22 angeordnete zweite Leiterbahnstruktur. Wie in der Figur 3 ist jeweils ein Thermoübergangsbereich 16 über jeweils eine elektrische Leiterbahn 14 mit einem Thermoübergangsbereich 18 verbunden, wobei alle elektrischen Leiter 14 und Thermoübergangsbereiche 16 und 18 aus einem Metall bestehen, welches sich von dem in der ersten Ebene 20 angeordneten Metall unterscheidet.

Aus den Figuren 5 und 6 sind die übereinander angeordneten Leiterbahnstrukturen, d.h. die Schichtstruktur der Figuren 3 und 4 ersichtlich. Die Thermoübergangsbereiche 16 und 18 der ersten 20 und zweiten 22 Ebene sind deckungsgleich übereinander angeordnet und weisen die gleiche Form auf, wobei in den Thermoübergangsbereichen 16 und 18 jeweilige Thermoübergänge zwischen den ersten 12 und zweiten 14 elektrischen Leitern gebildet werden. Die ersten 12 und zweiten 14 elektrischen Leiter bzw. die Thermoübergangsbereiche 16 und 18 sind derart angeordnet, dass sie elektrisch in Reihe liegen.

Zusätzlich ist zwischen der ersten 20 und der zweiten 22 Ebene eine elektrisch isolierende Schicht 21 angeordnet. Die elektrisch isolierende Schicht 21 ist bei den Thermoübergangsbereichen 16 und 18 durchbrochen, um die Thermoübergänge zwischen den ersten 12 und zweiten 14 elektrischen Leitern zu ermöglichen. Durch die elektrisch isolierende Schicht 21 können die ersten 12 und zweiten 14 elektrischen Leiter überlappend angeordnet werden, wodurch möglichst viele Thermoübergänge auf der Messfläche platzierbar sind. Insbesondere sind die Thermoübergangsbereiche 16 und 18 in den Ebenen 20 und 22 möglichst homogen verteilt. Dadurch können punktuelle Temperaturerhöhungen mit hoher Wahrscheinlichkeit erfasst werden. Durch die Anordnung der unterschiedlichen Leiter 12 und 14 in den beiden Schichten 20 und 22 wird insbesondere auf einfache Weise eine Aufteilung der Thermoübergänge 16 und 18 in zwei räumlich getrennte Gruppen ermöglicht, d.h. in den Figuren 3 bis 6 die Gruppierung der Thermoübergänge 16 nach links und der Thermoübergänge 18 nach rechts.

Nachfolgend wird die Herstellung der in den Figuren 5 und 6 schematisch gezeigten dreidimensionalen Schichtstruktur beispielhaft an Hand eines optischen Lithographie-Verfahrens beschrieben. Es sind jedoch auch andere Herstellungsverfahren denkbar.

Das Verfahren besteht im Wesentlichen aus drei Fertigungsabschnitten, in denen nachfolgend die erste Metallstruktur 12, 16, 18 in der ersten Ebene 20 (siehe Figur 3), die elektrisch isolierende Schicht 21 (siehe Figur 6) und die zweite Metallstruktur 14, 16, 18 in der zweiten Ebene 22 (siehe Figur 5) beschichtet werden.

Zur Herstellung der in der Figur 3 gezeigten ersten Metallstruktur 12, 16, 18 wird eine über einer dünnen Filmlackschicht angeordnete Maske belichtet. Die Maske weist die Struktur der in der Figur 3 schwarz gezeichneten Bereiche auf. Anschließend erfolgt eine Entwicklung der Filmlackschicht, wodurch die belichteten Bereiche 12, 16, 18 der Filmlackschicht entfernt werden. Dadurch bleiben nur die in der Figur 3 schwarz gezeichneten Bereiche übrig. Anschließend wird die erste Metallschicht aufgedampft. Danach wird die verbliebene Filmlackschicht entfernt, so dass die erste Metallstruktur 12, 16, 18 (wie in der Figur 3 weiß gezeigt) erhalten wird.

In einem nächsten Fertigungsschritt wird die elektrisch isolierende Schicht 21 auf die in der Figur 3 gezeigte erste Metallstruktur 12, 16, 18 aufgebracht. Danach werden in der elektrisch isolierenden Schicht 21 an den Thermoübergangsbereichen 16 und 18 Aussparungen ausgebildet. Die Aussparungen können beispielsweise in die elektrisch isolierende Schicht 21 geätzt oder mit einem optischen Lithographie-Verfahren gebildet werden.

Anschließend wird die in der Figur 4 gezeigte zweite Metallstruktur 14, 16, 18 auf die elektrisch isolierende Schicht 21 aufgebracht. Dies kann ebenfalls mit dem optischen Lithographie-Verfahren, wie zum Herstellen der in der Figur 3 gezeigten ersten Metallstruktur 12, 16, 18 verwendet, erfolgen. Dabei wird eine andere Maske verwendet, welche die Struktur des in der Figur 4 schwarz gezeichneten Bereichs aufweist. Beim Aufbringen bzw. Aufdampfen der zweiten Metallschicht 14, 16, 18 auf die elektrisch isolierende Schicht 21 gelangt das zweite Metall durch die in der elektrischen Isolierschicht 21 gebildeten Aussparungen mit dem ersten Metall in Verbindung. Dadurch werden die Thermoübergänge 16 und 18 gebildet.

Folglich wird durch das vorstehend beschriebene Herstellungsverfahren eine Metallschichtstruktur bereitgestellt, bei der unterschiedliche Metallschichten im Wesentlichen in zwei parallelen Ebenen 20 und 22 angeordnet sind, und elektrische Verbindungen zwischen den unterschiedliche Metallschichten im Wesentlichen in Ebenen liegen, die senkrecht zu den parallelen Ebenen 20 und 22 sind. So kann mit nur geringem Fertigungsaufwand eine Reihenschaltung von abwechselnden ersten 12 und zweiten 14 elektrischen Leitern realisiert werden.

Es ist auch denkbar, dass mehrere Schichtstrukturen 20, 21, 22 übereinander liegen, so dass mehrere in elektrischer Reihe verbundene Thermoübergänge 16, 18 in verschiedenen Ebenen übereinander angeordnet sind. Insbesondere kann zur genaueren Lokalisierung einer Temperaturerhöhung zwischen mindestens zwei in elektrischer Reihe verbundener Thermoübergänge mindestens ein Thermoübergang einer anderen Reihenschaltung angeordnet sein. Entsprechend kann durch Erhöhung der Anzahl von übereinander angeordneten Reihenschaltungen die Genauigkeit der Lokalisierung von Temperaturerhöhungen verbessert werden.

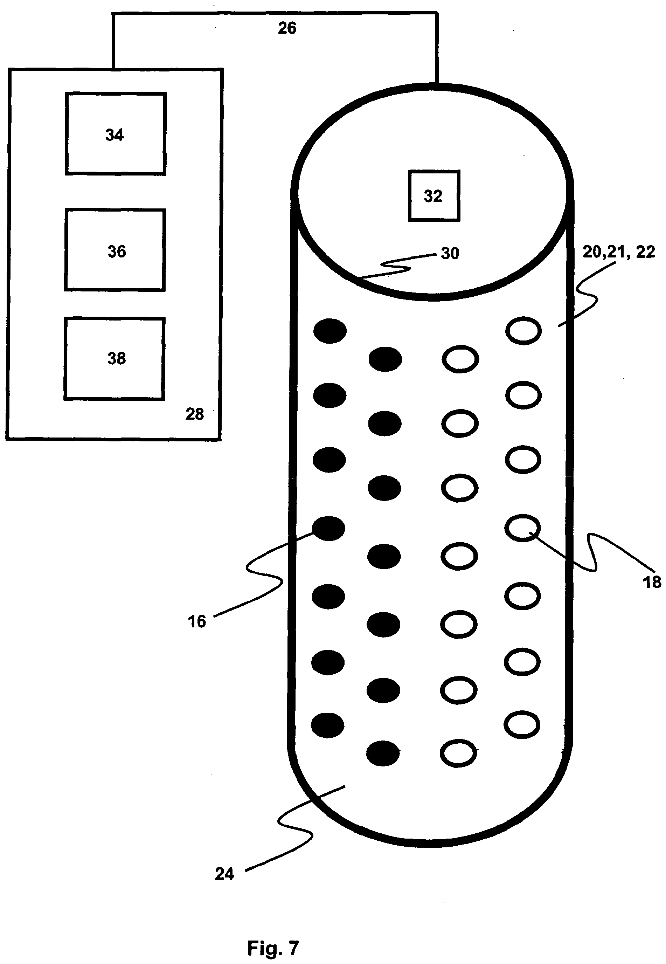

Die Figur 7 zeigt eine schematische Darstellung eines ersten Ausführungsbeispiels eines erfindungsgemäßen Speisenröhrensensors 24. Der Speisenröhrensensor 24 ist über eine Verbindungsleitung 26 mit einer Auswertungseinheit 28 verbunden. Bei der Verbindungsleitung 26 handelt es sich um ein Kabel, über welches der Speiseröhrensensor 24 in die Speiseröhre eingeführt bzw. wieder aus dieser herausgezogen werden kann.

Der Speiseröhrensensor 24 weist einen flächigen Körper 30 auf, welcher die Messfläche des Speiseröhrensensors 24 bildet. Der flächige Körper 30 hat eine Kreiszylinderform und besteht aus Kunststoff. Die vorliegende Erfindung ist auf die Form eines Zylinders beschränkt, aber jedoch nicht auf die Form eines Kreiszylinders und das Material Kunststoff beschränkt. Andere zylindrische Formen, und andere Materialien sind denkbar. Der in den Figuren 5 und 6 gezeigte Temperaturfühler 16, 18, 20, 21, 22 wurde gebogen und auf den flächigen Körper 30 geklebt.

Zusätzlich zu den Thermoübergängen 16 und 18 weist der Speiseröhrensensor 24 einen Temperatursensor 32 auf. Der Temperatursensor 32 misst die Temperatur und liefert Temperaturmesswerte an die Auswertungseinheit 28. Zusätzlich werden Spannungsänderungsmesswerte des Temperaturfühlers 16, 18, 20, 21, 22 der Auswertungseinheit 28 zugeführt.

Die Auswertungseinheit 28 weist eine elektrische Schaltung 34, eine Temperaturanzeige 36 und eine Warnvorrichtung 38 auf. Die elektrische Schaltung 34 bildet absolute Beträge der Spannungsänderungsmesswerte und addiert diese zu den Temperaturmesswerten des Temperatursensors 32. Die von der elektrischen Schaltung 34 bestimmten Werte werden der Anzeigevorrichtung 36 zugeführt und von dieser angezeigt. Überschreiten von der elektrischen Schaltung 34 bestimmte Werte einen vorbestimmten Grenzwert, so gibt die Warnvorrichtung 38 ein Warnsignal aus. Es ist auch denkbar, dass die Auswertungseinheit mit einer thermochirurgischen Vorrichtung (nicht gezeigt) verbunden ist und bei einer einen Grenzwert überschreitenden Temperaturerhöhung automatisch die thermochirurgische Vorrichtung abschaltet. Dadurch können unerwünschte Gewebeüberhitzungen vermieden werden.

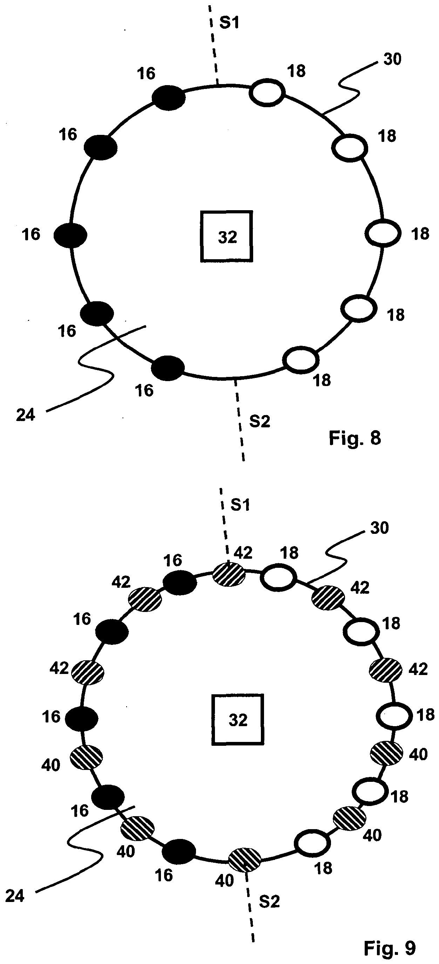

Die Figur 8 zeigt schematisch in einer Ansicht von oben den Speiseröhrensensor 24 der Figur 7. Wie aus der Figur 8 ersichtlich ist, kann mit Hilfe einer gruppierten Anordnung bzw. Aufteilung der Thermoübergänge 16 und 18 erkannt werden, in welcher Hälfte des Speiseröhrensensors 24 eine Temperaturänderung auftritt. Dazu kann der Speisenröhrensensor 24 beispielsweise durch drehen des Kabels 26 unter Zuhilfenahme eines Endoskops (nicht gezeigt) in eine gewünschte Ausrichtung gebracht werden.

Probleme können sich jedoch ergeben, falls eine punktuelle (d.h. eine geringflächige) Temperaturerhöhung in einem der Bereiche S1 oder S2 auftritt, d.h. in einem Bereich zwischen den Thermoübergangsgruppen 16 und 18. In diesem Fall können sich die von dem Thermoübergängen 16 und 18 erzeugten Spannungsänderungen zu Null aufheben, wodurch eine Temperaturerhöhung nicht erfasst werden kann.

Zur Lösung dieses Problems kann eine zweite Reihenschaltung von Thermopaaren vorgesehen werden. Die Figur 9 zeigt schematisch in einer Ansicht von oben ein zweites Ausführungsbeispiel eines Speiseröhrensensors 24 aufweisend zwei Reihenschaltungen von Thermopaaren.

Das Ausführungsbeispiel der Figur 9 unterscheidet sich von dem Ausführungsbeispiel der Figuren 7 und 8, in dem zwischen den Thermoübergängen 16 und 18 der ersten Reihenschaltung Thermoübergänge 40 und 42 einer zweiten Reihenschaltung angeordnet sind. Die Thermoübergänge 40 liefern bei einer gegebenen Temperaturerhöhung eine positive Spannungsänderung, wohingegen die Thermoübergänge 42 bei einer gegebenen Temperaturerhöhung eine negative Spannungsänderung liefern. Wie aus der Figur 9 ersichtlich, sind die mit kürzestem Abstand voneinander direkt benachbarten Thermoübergänge 16 und 18 um 90° versetzt zu den mit kürzestem Abstand voneinander direkt benachbarten Thermoübergängen 40 und 42 an der Messfläche 30 des Speiseröhrensensors 24 angeordnet. Durch diese Anordnung der Thermoübergänge 16, 18, 40, 42 werden vier Messbereiche definiert. Somit kann festgestellt werden, in welchem der vier Messbereiche eine Temperaturerhöhung auftritt. Durch eine Erhöhung der Anzahl von Reihenschaltungen kann die Anzahl von Messbereichen erhöht werden, wodurch eine noch genauere Lokalisierung von Temperaturerhöhungen möglich ist. Beispielsweise können bei drei übereinander angeordneten Reihenschaltungen die mit kürzestem Abstand voneinander direkt benachbarten Thermoübergänge um 60° versetzt zueinander auf der Messfläche angeordnet sein, so dass Temperaturerhöhungen in sechs Messbereichen lokalisiert werden können.

Erfolgt eine punktuelle Temperaturerhöhung in einem der Bereiche S1 oder S2, so wird diese Temperaturerhöhung durch die in den Bereichen S1 und S2 angeordneten Thermoübergänge 40 bzw. 42 der zweiten Reihenschaltung erfasst, wodurch auch bei sich gegenseitig aufhebenden Spannungsänderungen der Thermoübergänge 16 und 18 der ersten Reihenschaltung die punktuelle Temperaturerhöhung zuverlässig erfasst werden kann.

Die in den Figuren 1 bis 8 gezeigten Anordnungen der Thermoübergänge 16 und 18 auf der Messfläche sind nur beispielhaft. Eine Vielzahl verschiedener Anordnungen sind denkbar. So ist es beispielsweise bei dem in den Figuren 7 bis 9 gezeigten Speiseröhrensensoren 24 denkbar, dass eine Gruppe von Thermoübergängen 16 in der Mitte des Kreiszylinders 30 (d.h. bei dem Temperatursensor 32) angeordnet ist, wobei die Thermoübergänge 18 der weiteren Gruppe auf der Oberfläche des Kreiszylinders 30 liegen.

Des Weiteren bezogen sich die vorangegangenen Ausführungsbeispiele nur auf unerwünschte Gewebeüberhitzungen (d.h. Temperaturerhöhungen) bei der Thermochirurgie. Die vorliegende Erfindung kann jedoch auch bei einer Kältetherapie, insbesondere bei der sogenannten "Cryo-Technik", d.h. einer Ablation von lebendem Gewebe mit Hilfe von Kälte, eingesetzt werden. Entsprechend kann durch den erfindungsgemäßen Temperaturfühler eine unerwünschte Temperaturreduktion von lebendem Gewebe erfasst werden.

## Patentansprüche

1. Temperaturfühler (24) zur Temperaturmessung an oder in einem lebenden Körper, mit einer Vielzahl auf einer Messfläche (30) des Fühlers (24) verteilt angeordneter, elektrisch zu mindestens einer Reihenschaltung (10) verbundener Thermopaare, wobei in jeder Reihenschaltung von den Thermopaaren gebildete Thermoübergänge (16, 18) auf mindestens und vorzugsweise insgesamt zwei räumlich voneinander getrennte Gruppen aufgeteilt sind, derart, dass in jeder Gruppe die Thermoübergänge (16, 18) bei einer gegebenen Temperaturänderung eine jeweils gleichsinnige Spannungsänderung bewirken,
wobei jedes Thermopaar einen ersten und einen zweiten elektrischen Leiter (12, 14) aufweist, die an einem Thermoübergang miteinander verbunden sind,
wobei der Temperaturfühler einen Schichtenaufbau mit einer ersten Leiterschicht (20), in der eine erste metallische Leiterstruktur gebildet ist, einer zweiten Leiterschicht (22), in der eine zweite metallische Leiterstruktur gebildet ist, und einer zwischen den beiden Leiterschichten angeordneten elektrisch isolierenden Schicht (21) besitzt, wobei die Leiterstrukturen der beiden Leiterschichten aus unterschiedlichen Metallen bestehen,
wobei der erste Leiter (12) jedes Thermopaars von der ersten Leiterstruktur gebildet ist und der zweite Leiter (14) jedes Thermopaars von der zweiten Leiterstruktur gebildet ist und dass die elektrisch isolierende Schicht (21) Aussparungen besitzt, durch welche hindurch die ersten und zweiten Leiter (12, 14) der Thermopaare verbunden sind, wobei die Messfläche des Temperaturfühlers die Form eines Zylinders aufweist und zumindest ein Teil der Thermoübergänge auf der Mantelfläche des Zylinders angeordnet ist.

2. Temperaturfühler nach Anspruch 1, bei dem die Thermoübergänge (16, 18) im Wesentlichen homogen verteilt auf der Messfläche (30) angeordnet sind.

3. Temperaturfühler nach Anspruch 1 oder 2, bei dem die Thermoübergänge (16, 18) die Form von Sechsecken haben.

4. Temperaturfühler nach einem der vorhergehenden Ansprüche, bei dem die ersten Leiter (12) überlappungsfrei in der ersten Leiterschicht (20) und die zweiten Leiter (14) überlappungsfrei in der zweiten Leiterschicht (22) liegen.

5. Temperaturfühler nach Anspruch 4, bei dem sich die in der ersten Leiterschicht (20) liegenden ersten Leiter (12) und die in der zweiten Leiterschicht (22) liegenden zweiten Leiter (14) gegenseitig teilweise überlappen.

6. Temperaturfühler nach einem der vorhergehenden Ansprüche, bei dem die Thermopaare (16, 18, 40, 42) elektrisch zu mindestens zwei Reihenschaltungen verbunden sind.

7. Temperaturfühler nach Anspruch 6, bei dem zwischen mindestens zwei in elektrischer Reihe (10) verbundenen Thermoübergängen (16, 18) mindestens ein Thermoübergang (40, 42) einer anderen Reihenschaltung angeordnet ist.

8. Temperaturfühler nach Anspruch 6 oder 7, bei dem Thermopaare (16, 18, 40, 42) der mindestens zwei Reihenschaltungen gegenseitig teilweise überlappend auf der Messfläche (30) angeordnet sind.

9. Temperaturfühler nach Anspruch 6 oder 7, bei dem Thermopaare (16, 18, 40, 42) der mindestens zwei Reihenschaltungen gegenseitig überlappungsfrei auf der Messfläche (30) angeordnet sind.

10. Temperaturfühler nach einem der vorhergehenden Ansprüche, bei dem die ersten und zweiten Leiter (12, 14) und die Thermoübergänge (16, 18) auf einer flexiblen Leiterplatte angeordnet sind.

11. Temperaturfühler nach Anspruch 10, bei dem die flexible Leiterplatte auf die Messfläche des Temperaturfühlers aufgeklebt ist.

12. Temperaturfühler nach einem der vorhergehenden Ansprüche mit einer elektrischen Schaltung (34) zum Bilden eines absoluten Betrags eines Messwerts des Temperaturfühlers (24).

13. Temperaturfühler nach Anspruch 12 mit
einem Temperatursensor (32) zum Messen eines Temperaturwerts, wobei die elektrische Schaltung (34) den von dem Temperatursensor (32) gemessenen Temperaturwert und den absoluten Betrag des Messwerts zu addieren vermag.

14. Speiseröhrensensor (24) mit einem Temperaturfühler nach einem der vorhergehenden Ansprüche.

15. Speiseröhrensensor nach Anspruch 14, bei dem die Thermoübergänge (16, 18) einer Reihenschaltung auf zwei Gruppen aufgeteilt sind, die in jeweils einer Zylinderhälfte des Speiseröhrensensors liegen.

## Claims

1. A temperature sensor (24) for measuring the temperature on or in a living body, with a plurality of thermocouples arranged in a distributed manner on a measurement surface (30) of the sensor (24) and electrically connected to create at least one series circuit (10), wherein thermojunctions (16, 18) formed by the thermocouples in each series circuit are divided into at least two, and preferably into a total of two, spatially separate groups such that in each group, when there is a given change in temperature, the thermojunctions (16, 18) cause a corresponding change in voltage,
wherein each thermocouple comprises a first and a second electrical conductor (12, 14) which are connected with one another at a thermojunction,
wherein the temperature sensor has a layered structure with a first conductor layer (20) in which a first metallic conductor structure is formed, a second conductor layer (22) in which a second metallic conductor structure is formed, and an electrically insulating layer (21) which is disposed between the two conductor layers, wherein the conductor structures of the two conductor layers consist of different metals,
wherein the first conductor (12) of each thermocouple is formed by the first conductor structure, and the second conductor (14) of each thermocouple is formed by the second conductor structure, and the electrically insulating layer (21) comprises recesses through which the first and the second conductors (12, 14) of the thermocouples are connected,
wherein the measurement surface of the temperature sensor has a cylindrical shape, and at least a part of the thermocouples are arranged on the circumferential surface of the cylinder.

2. The temperature sensor in accordance with Claim 1, wherein the thermojunctions (16, 18) are essentially homogeneously distributed on the measurement surface (30).

3. The temperature sensor in accordance with Claim 1 or 2, wherein the thermojunctions (16, 18) have the shape of hexagons.

4. The temperature sensor in in accordance with any of the foregoing claims, wherein the first conductors (12) are disposed with no overlap in the first conductor layer (20) and the second conductors (14) are disposed with no overlap in the second condutor layer (22).

5. The temperature sensor in accordance with Claim 4, wherein the first conductors (12) disposed in the first conductor layer (20) and the second conductors (14) disposed in the second conductor layer (22) partially mutually overlap.

6. The temperature sensor in accordance with any of the foregoing claims, wherein the thermocouples (16, 18, 40, 42) are electrically connected to create at least two series circuits.

7. The temperature sensor in accordance with claim 6, wherein at least one thermojunction (40, 42) from another series circuit is arranged between at least two thermojunctions (16, 18) connected in electrical series (10).

8. The temperature sensor in accordance with claim 6 or 7, wherein thermocouples (16, 18, 40, 42) of the at least two series circuits are arranged on the measurement surface (30) partially mutually overlapping.

9. The temperature sensor in accordance with Claim 6 or 7, wherein thermocouples (16, 18, 40, 42) of the at least two series circuits are arranged on the measurement surface (30) without mutual overlap.

10. The temperature sensor in accordance with one of the foregoing claims, wherein the first and the second conductors (12, 14) and the thermojunctions (16, 18) are arranged on a flexible circuit board.

11. The temperature sensor in accordance with Claim 10, wherein the flexible circuit board is glued on the measurement surface of the temperature sensor.

12. The temperature sensor in accordance with one of the foregoing claims, comprising an electrical circuit (34) for obtaining an absolute value of a measured value of the temperature sensor (24).

13. The temperature sensor in accordance with Claim 12, comprising a temperature sensor (32) for measuring a temperature value, wherein the electrical circuit (34) is able to add the temperature value measured by the temperature sensor (32) and the absolute value of the measured value.

14. An esophageal sensor (24) with a temperature sensor in accordance with any of the foregoing claims.

15. The esophageal sensor in accordance with Claim 14, wherein the thermojunctions (16, 18) of a series circuit are divided into two groups, each of which being located in a cylinder half of the esophageal sensor.

## Revendications

1. Sonde de température (24) pour mesurer la température sur ou dans un corps vivant, comportant une pluralité de thermocouples répartis sur une surface de mesure (30) de la sonde (24) et reliés électriquement à au moins un circuit série (10), dans laquelle, dans chaque circuit série, les jonctions thermiques (16, 18) formées par les thermocouples sont réparties en au moins et de préférence en un total de deux groupes spatialement séparés, de telle sorte que, dans chaque groupe, les jonctions thermiques (16, 18) provoquent une variation de tension dans le même sens pour une variation de température donnée,
dans laquelle chaque thermocouple présente un premier et un second conducteur électrique (12, 14) connectés ensemble à une jonction thermique,
dans laquelle la sonde de température a une structure en couches avec une première couche conductrice (20), dans laquelle une première structure conductrice métallique est formée, une seconde couche conductrice (22), dans laquelle une seconde structure conductrice métallique est formée, et une couche électriquement isolante (21) disposée entre les deux couches conductrices, les structures conductrices des deux couches conductrices étant constituées de métaux différents,
dans laquelle le premier conducteur (12) de chaque thermocouple est formé par la première structure conductrice et le second conducteur (14) de chaque thermocouple est formé par la seconde structure conductrice et la couche électriquement isolante (21) présente des évidements à travers lesquels les premier et second conducteurs (12, 14) des thermocouples sont connectés,
dans laquelle la surface de mesure de la sonde de température présente la forme d'un cylindre et au moins une partie des jonctions thermiques est disposée sur la surface latérale du cylindre.

2. Sonde de température selon la revendication 1, dans laquelle les jonctions thermiques (16, 18) sont réparties de manière sensiblement homogène sur la surface de mesure (30).

3. Sonde de température selon la revendication 1 ou 2, dans laquelle les jonctions thermiques (16, 18) se présentent sous la forme d'hexagones.

4. Sonde de température selon l'une des revendications précédentes, dans laquelle les premiers conducteurs (12) sont placés sans se chevaucher dans la première couche conductrice (20) et les seconds conducteurs (14) sont placés sans se chevaucher dans la seconde couche conductrice (22).

5. Sonde de température selon la revendication 4, dans laquelle les premiers conducteurs (12) placés dans la première couche conductrice (20) et les seconds conducteurs (14) placés dans la seconde couche conductrice (22) se chevauchent partiellement.

6. Sonde de température selon l'une des revendications précédentes, dans laquelle les thermocouples (16, 18, 40, 42) sont connectés électriquement en au moins deux circuits série.

7. Sonde de température selon la revendication 6, dans laquelle au moins une jonction thermique (40, 42) d'un autre circuit série est disposée entre au moins deux jonctions thermiques (16, 18) connectées en un circuit électrique série (10).

8. Sonde de température selon la revendication 6 ou 7, dans laquelle les thermocouples (16, 18, 40, 42) desdits au moins deux circuits série sont disposés de manière à se chevaucher partiellement sur la surface de mesure (30).

9. Sonde de température selon la revendication 6 ou 7, dans laquelle les thermocouples (16, 18, 40, 42) desdits au moins deux circuits série sont disposés sans se chevaucher sur la surface de mesure (30).

10. Sonde de température selon l'une des revendications précédentes, dans laquelle les premiers et seconds conducteurs (12, 14) et les jonctions thermiques (16, 18) sont disposés sur une carte de circuit imprimé flexible.

11. Sonde de température selon la revendication 10, dans laquelle la carte de circuit imprimé flexible est collée sur la surface de mesure de la sonde de température.

12. Sonde de température selon l'une des revendications précédentes, comprenant un circuit électrique (34) pour former une valeur absolue d'une valeur mesurée par la sonde de température (24).

13. Sonde de température selon la revendication 12, comprenant un capteur de température (32) pour mesurer une valeur de température, le circuit électrique (34) étant capable d'additionner la valeur de température mesurée par le capteur de température (32) et la valeur absolue de la valeur mesurée.

14. Capteur d'oesophage (24) comprenant une sonde de température selon l'une des revendications précédentes.

15. Capteur d'oesophage selon la revendication 14, dans lequel les jonctions thermiques (16, 18) d'un circuit série sont réparties en deux groupes qui sont placés chacun dans un demi-cylindre du capteur d'oesophage.
